# EUROPEAN PATENT APPLICATION

(11) **EP 3 064 225 A1**
(43) Date of publication of application: **07.09.2016**
(21) Application number: 16158115.2
(22) Date of filing: 01.03.2016
(51) Int. Cl.: A61L 2/22, A61L 9/14, B05B 3/10

(54) **METHOD AND DEVICE FOR CLEANING AND/OR DISINFECTING THE AIR OF ENCLOSED AND/OR CONFINED SPACES INTENDED TO BE USED BY PERSONS AND/OR ANIMALS**

(30) Priority: 02.03.2015 IT MI20150307
(71) Applicant: Menotti, Dario, 24043 Caravaggio (BG) (IT)
(72) Inventor: Menotti, Dario, 24043 Caravaggio (BG) (IT)
(74) Representative: Modiano, Micaela Nadia

(57) **Abstract**

A method for cleaning and/or disinfecting the air of enclosed and/or confined spaces intended to be used by persons and/or animals, the method comprising:
- a step of dispensing, into the air of a space to be treated, an atomized liquid;
- a step of waiting for the atomized liquid to fall;
- a step of collecting the fallen liquid from the surfaces of the space into which it was dispensed.

## Description

The present invention relates to a method and a device for cleaning and/or disinfecting the air of enclosed and/or confined spaces intended to be used by persons and/or animals. More specifically, the present invention relates to a method and to a device which are capable of purifying the air of pollutant substances, in particular small particulates, but also pollen, microorganisms or other pollutant agents which are found dispersed in suspension in the air of enclosed and/or confined spaces of any size where persons or animals usually stop in, pass through, or live, such as for example trains, dwellings, hospitals, nursing homes, offices, industrial environments etc.

As is known, the air we breathe is often polluted by particles and microorganisms which we inhale by breathing in and which can have harmful consequences for our health.

It is likewise known that most people spend many hours in enclosed spaces in which the exchange of air is greatly reduced, particularly in winter. Besides, changing the air frequently does not necessarily improve the quality of the air since the outside air is not necessarily better than the air inside the space.

In many cases the need is therefore felt to purify the air by eliminating, as far as possible, the pollutant substances present therein.

This need is particularly felt in healthcare places and medical studios, where persons temporarily stay who are affected by diseases and who are particularly vulnerable to infection by viruses and bacteria, and also to particulates or allergens, and who may, in turn, disperse substances or microorganisms into the air of the space, which may be harmful for the staff or for the other patients or for visitors.

This need is also felt however for places that are frequented by many persons, such as for example in communities, on means of public transportation, in work spaces, but also in dwellings.

To meet the need to purify the air of spaces, nowadays air treatment plants are used, which however require costly installations which must almost always be done during construction or renovation of the building. Furthermore, these plants, in order to be efficient, require frequent maintenance and high running costs.

Finally, these plants can be adopted only for large spaces or in buildings with a large number of spaces to be treated.

Very often, the need to clean and disinfect a space is met only for the surfaces of the space that can be most easily reached, such as for example the floors, the walls, the windows, the furniture, with no effect on the air contained in these spaces.

In the sector of air treatment for special environments, such as for example operating rooms, techniques and apparatuses are known for disinfecting a space and the objects contained therein. One of these techniques is disclosed, for example, in US 2011/123394 A1 and is based on the heating and subsequent dispersion of a mixture of water and hydrogen peroxide in the air of the space to be disinfected. The mixture of water and hydrogen peroxide is recovered, after such mixture has carried out its disinfectant action, by dehumidifying the air of the treated space.

A technique of the type disclosed in US 2011/123394 A1 necessarily requires the availability of an air treatment plant in order to carry out the recovery of the mixture that has been dispensed in the environment.

Furthermore, a technique of this kind, owing to the fact that the mixture used is recovered by aspiration and dehumidification, offer scant effectiveness in the physical abatement of pollutant substances dispersed in the air, such as for example particulates, which are difficult to capture by way of aspiration.

Furthermore, a technique of the type disclosed in US 2011/123394 A1 is costly to implement owing to the equipment it requires.

The aim of the present invention is to solve the above mentioned drawback, by devising a method for cleaning and/or disinfecting the air of enclosed and/or confined spaces intended to be used by persons and/or animals with which it is possible to abate pollutants simply and at low cost.

Within this aim, an object of the invention is to provide a method that, in order to be carried out, does not necessarily require costly installations.

Another object of the invention is to provide a method that is simple and rapid to carry out.

Another object of the invention is to provide a device, practical in use, which can be used in order to carry out the method in question.

Another object of the invention is to provide a device that requires reduced maintenance operations and which offers the highest guarantees of safety and reliability in use.

This aim and these and other objects which will become better apparent hereinafter are achieved by a method for cleaning and/or disinfecting the air of enclosed and/or confined spaces, intended to be used by persons and/or animals, characterized in that it comprises:
- a step of dispensing, into the air of a space to be treated, an atomized liquid;
- a step of waiting for the atomized liquid to fall;
- a step of collecting the fallen liquid from the surfaces of the space into which it was dispensed.

In order to carry out the method according to the invention, a device is preferably used which is characterized in that it comprises a storage tank for storing a liquid, which is provided with means for pressurizing said liquid and is connected, by means of a connecting duct, to an atomizer which is adapted to dispense said liquid in atomized form.

Further characteristics and advantages of the invention will become better apparent from the detailed description that follows of a preferred, but not exclusive, embodiment of the method according to the invention and of a device for its execution, which is illustrated for the purposes of nonlimiting example in the accompanying drawings wherein:

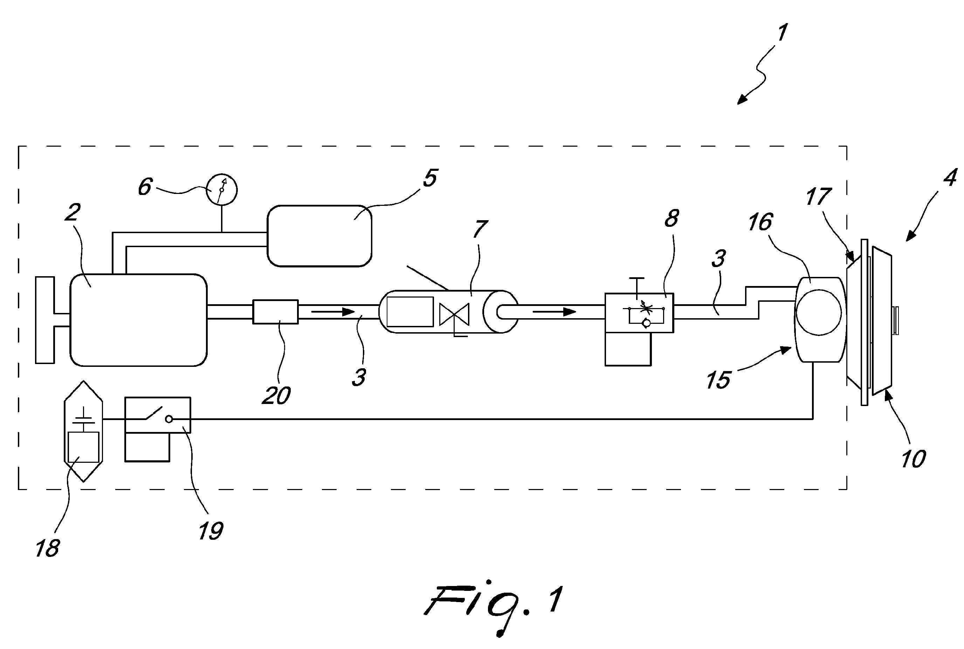

Figure 1 schematically illustrates the device for carrying out the method according to the invention;

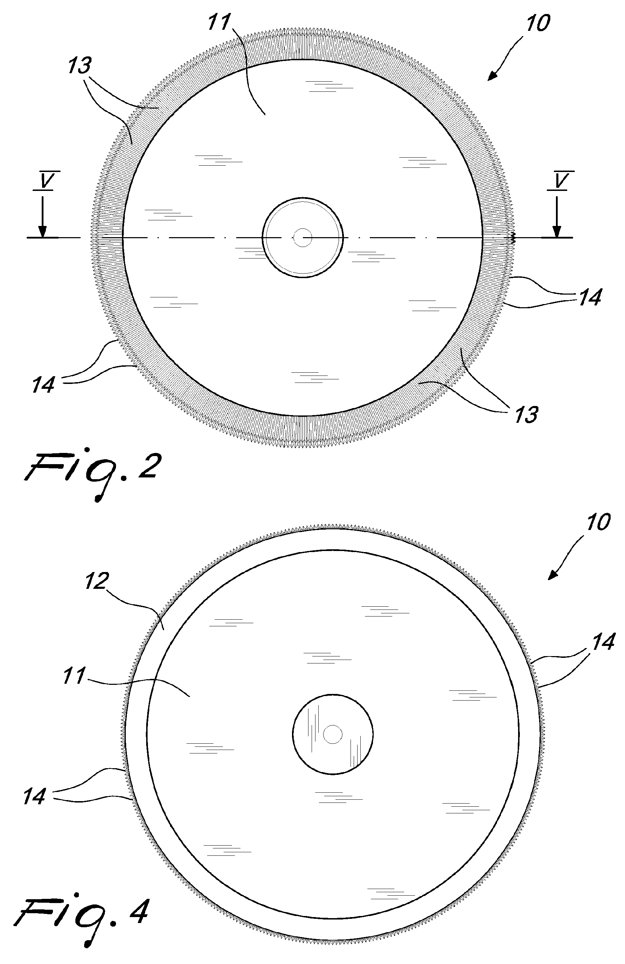

Figure 2 is a plan view from above of a component of the device;

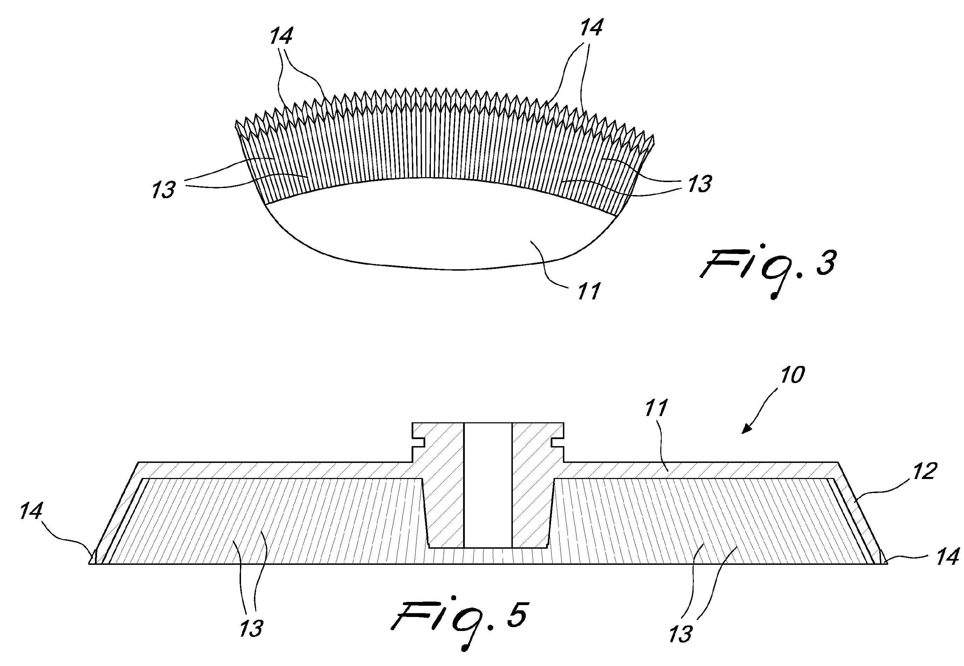

Figure 3 is an enlarged view of a detail of Figure 2;
Figure 4 is a plan view from below of the same component of Figure 2;
Figure 5 is a cross-sectional view of Figure 2 taken along the line V-V;
Figure 6 is an enlarged view of a detail of Figure 5;
Figure 7 is a cross-sectional view of Figure 6 taken along the line VII-VII;
Figure 8 is a cross-sectional view of Figure 6 taken along the line VIII-VIII;
Figure 9 schematically illustrates a portion of the device, in a side elevation view, with some elements omitted for the sake of simplicity.

With reference to the figures, a device that can be used in order to carry out the method according to the invention, generally designated by the reference numeral 1, comprises a storage tank 2, which is adapted to contain the liquid to be used and is provided with means for pressurizing the liquid. The storage tank 2 is connected, by way of a connecting duct 3, to an atomizer 4 which is adapted to dispense the liquid in atomized form.

The means for pressurizing the liquid contained inside the storage tank 2 can be constituted by a conventional manually actuated pump or by a mechanically-actuated pump, also conventional. Such pump is preferably integrated in the storage tank 2 and is not shown in the figures.

Preferably, a balance tank 5 is provided which is connected to the storage tank 2. The function of the balance tank 5 is to maintain the liquid contained in the storage tank 2 at a preset pressure, preferably about 1.5 bar.

Along the duct that connects the balance tank 5 to the storage tank 2, there is a gauge 6 which makes it possible to control the pressure present inside the storage tank 2 so that, if the pressure should drop below the desired value, the operator can intervene, by way of the pump, in order to restore the pressure to the desired value.

Arranged along the connecting duct 3 is a manually-actuated opening-closing faucet 7.

Upstream of the faucet 7, along the connecting duct 3, there is conveniently a filter 20 for retaining any impurities present in the liquid originating from the storage tank 2.

Furthermore, between the faucet 7 and the atomizer 4, there is a one-way valve for adjusting the flow 8 of the liquid that transits from the storage tank 2 to the atomizer 4.

The atomizer 4 comprises a nozzle 9 which is connected to the connecting duct 3 and faces a disk-like element 10 which has a base 11 with a circular plan shape and a perimetric edge 12 which extends from the base 11 and is shaped like a conical surface that widens progressively on the opposite side with respect to the base 11. The perimetric edge 12 is provided, on its inner surface, with a plurality of teeth 13 which extend along the directrices of the conical surface.

Conveniently, the perimetric edge 12 has, proximate to its end that lies opposite the base 11, a plurality of teeth 14 on its outer surface as well.

The nozzle 9 faces toward the inner surface of the disk-like element 10 and means 15 are provided for actuating the disk-like element 10 with a rotary motion about its own axis.

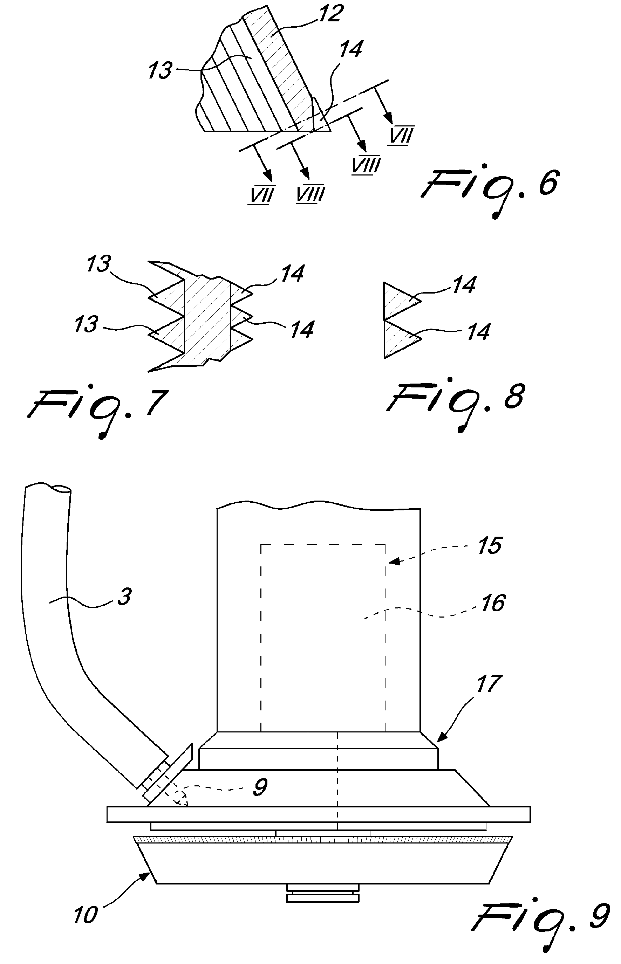

More specifically, the actuation means 15 are constituted by an electric motor 16, which is accommodated inside a supporting element 17 and is connected, with its shaft, to the disk-like element 10. The electric motor 16 can be connected to the mains electricity supply, or, as illustrated, it can be powered by batteries 18 and actuated by way of a switch 19. Figure 9 schematically shows the supporting element 17 which contains the electric motor 16. The supporting element 17 faces toward the concave side of the disk-like element 10 and, between the supporting element 17 and the disk-like element 10, there is a space through which the liquid atomized by the action of the atomizer 4 is diffused outwardly.

Preferably, the diameter of the nozzle 9 is comprised substantially between 0.65 mm and 0.75 mm.

The several elements described above, which comprise the device for carrying out the method according to the invention, can be mounted on a supporting structure which is contoured so as to be easily taken up by an operator or in any case easily transportable, or indeed provided with wheels in order to facilitate the movement thereof.

Operation of the device described above, in carrying out the method according to the invention, is the following.

The air of the confined space that it is desired to clean and/or disinfect is preferably subjected to analysis by measuring the concentration of pollutant substances present in such air before the treatment.

The liquid to be used to treat the environment for which it is desired to carry out the cleaning and/or the disinfection of the air is introduced into the storage tank 2 and is put under pressure by way of the pump. The liquid to be used can be constituted simply by water or by a solution containing a disinfectant and/or sanitizing substance, according to the pollutants that have to be eliminated from the air.

Acting on the switch 19 actuates the electric motor 16 which turns the disk-like element 10 about its own axis.

At this point, by opening the faucet 7, the nozzle 9 is fed with the liquid originating from the storage tank 2 and the nozzle 9 projects the liquid onto the inner surface of the disk-like element 10 which is in rotation. The impact of the liquid against the toothed surface of the disk-like element 10 causes the atomization of the liquid which is diffused into the air of the environment.

The pressure for feeding the liquid and the dimensions of the nozzle 9, as well as the speed of the disk-like element 10, are designed so as to obtain an atomization of the liquid with droplets of diameter preferably comprised between 140 µm and 200 µm. The diameter of the droplets of micronized liquid is chosen as a function of the particles to be abated and can be varied in a simple manner by varying the diameter of the nozzle 9, for example by substituting the nozzle 9 with one of a different diameter according to the operation to be carried out.

By way of the one-way valve for adjusting the flow 8, it is possible to vary the diameter of the cloud of micronized liquid as a function of the size of the space to be treated.

The atomized liquid, emitted by the atomizer 4, descends slowly onto the floor and onto any other objects that are present in the environment, taking the pollutant substances present in the air with it. Once the time necessary in order to allow the atomized liquid to be completely deposited has elapsed, it is sufficient to carry out its removal from the surfaces of the environment, by way of a simple cleaning operation, for example by way of a cloth or the like, in order to remove the pollutant substances that have been captured and dragged down by the atomized liquid.

Preferably, at the end of the treatment, the analysis of the air of the treated space is repeated, again measuring the concentration of residual pollutant substances so as to assess the efficacy of the treatment carried out.

In practice, the method according to the invention makes it possible to carry out a true washing of the air of an environment, removing pollutant substances, like particulates, but also microorganisms like bacteria or germs or allergens that may be present in the air, thus performing an operation not just of cleaning, but also of disinfection.

It should be noted that the method according to the invention does not necessarily require the use of chemical substances. As demonstrated by practical experimentation, even by using only water it is possible to obtain a marked reduction of the pollutant substances, in particular of particulates, that are present in the air of the treated space. The micro-droplets projected into the air succeed, in fact, in trapping the pollutants dispersed in the air and in bringing them to the ground, in this manner cleaning the air. The final operation of manually cleaning what is deposited on the ground, and that is to say the mixture of water and pollutant substances, completes the method of cleaning the air according to the invention.

In practice it has been found that the method according to the invention fully achieves the set aim in that it makes it possible to eliminate pollutant substances from the air of enclosed and/or confined spaces intended to be used by persons and/or animals simply and at low cost.

Another advantage of the method according to the invention is that it can be carried out in combination with normal cleaning operations without requiring complex installations or lengthy execution times.

Another advantage of the method according to the invention is that it has no negative impact on humans and animals that frequent the space subjected to treatment, or on the environment in general. Repeated application over time of the method according to the invention markedly improves the quality of the air with beneficial effects on the health of the users of the treated space.

It should be noted that the method according to the invention radically changes the concept of cleaning enclosed and/or confined spaces in that it also takes account of the air of these spaces, and therefore it lends itself to a quantitative system for calculating the material to be cleaned by the cubic meter instead of by the square meter.

The method, and the device for its execution, thus conceived, are susceptible of numerous modifications and variations, all of which are within the scope of the appended claims; moreover, all the details may be substituted by other, technically equivalent elements.

In practice the materials employed, and the dimensions, may be any according to requirements and to the state of the art.

The disclosures in Italian Patent Application No. MI2015A000307 (102015902335350) from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for cleaning and/or disinfecting the air of enclosed and/or confined spaces, intended to be used by persons and/or animals, **characterized in that** it comprises:
- a step of dispensing, into the air of a space to be treated, an atomized liquid;
- a step of waiting for the atomized liquid to fall;
- a step of collecting the fallen liquid from the surfaces of the space into which it was dispensed.

2. The method according to claim 1, **characterized in that**, before said step of dispensing an atomized liquid, a measurement is carried out of the concentration of the pollutant substances in the air of the space to be treated.

3. The method according to claims 1 and 2, **characterized in that**, after said step of collecting the fallen liquid, another measurement is carried out of the concentration of the pollutant substances in the air of the treated space.

4. The method according to claim 1, **characterized in that** said liquid is constituted by water.

5. The method according to claim 1, **characterized in that** said liquid comprises at least one disinfectant and/or sanitizing substance.

6. The method according to one or more of the preceding claims, **characterized in that** the diameter of the droplets of said atomized liquid is substantially comprised between 140 µm and 200 µm.

7. A device for cleaning and/or disinfecting the air of enclosed and/or confined spaces intended to be used by persons and/or animals, **characterized in that** it comprises a storage tank (2) for storing a liquid, which is provided with means for pressurizing said liquid and is connected, by means of a connecting duct (3), to an atomizer (4) which is adapted to dispense said liquid in atomized form.

8. The device according to claim 7, **characterized in that** it comprises a balance tank (5) which is connected to said storage tank (2) in order to maintain a preset pressure in said storage tank (2).

9. The device according to one or more of claims 7 and 8, **characterized in that** an opening-closing faucet (7) is arranged along said connecting duct (3).

10. The device according to one or more of claims 7-9, **characterized in that** a one-way valve for adjusting the flow (8) of said liquid is arranged between said opening-closing faucet (7) and said atomizer (4).

11. The device according to one or more of claims 7-10, **characterized in that** said atomizer (4) comprises a nozzle (9) which is connected to said connecting duct (3) and faces a disk-like element (10) which has a base (11) with a circular plan shape and a perimetric edge (12) which extends from said base (11) and is shaped like a conical surface that widens progressively on the opposite side with respect to said base (11), said perimetric edge (12) having, on its inner surface, a plurality of teeth (13) which extend along the directrices of the conical surface, said nozzle (9) facing the inner surface of said disk-like element (10), means (15) for actuating said disk-like element (10) with a rotary motion about its own axis being provided.

12. The device according to one or more of claims 7-11, **characterized in that** said perimetric edge (12) has, proximate to its end that lies opposite said base (11), a plurality of teeth (14) on its outer surface as well.
